# EUROPEAN PATENT SPECIFICATION

(11) **EP 3 569 177 B1**
(45) Date of publication and mention of the grant of the patent: **24.03.2021**
(21) Application number: 19185041.1
(22) Date of filing: 08.08.2014
(51) Int. Cl.: A61B 18/14, A61M 25/10, A61B 18/00

(54) **ELECTROSURGICAL DEVICE**
ELEKTROCHIRURGISCHE VORRICHTUNG
DISPOSITIF ÉLECTROCHIRURGICAL

(30) Priority: 09.08.2013 US 201313963202
(43) Date of publication of application: 20.11.2019
(62) Divisional of application: 14756170.8
(73) Proprietor: Medtronic Xomed, Inc., Jacksonville, FL 32216-0980 (US)
(72) Inventor: BLOOM, Eliot F., Hopkinton New Hampshire 03229 (US)
(74) Representative: Dehns

(56) References cited:
- US-A1- 2013 184 702
- US-B1- 6 514 250

## Description

### Background

The human body includes a number of internal body lumens, passageways, and cavities, many of which have an inner lining or layer. These inner layers can be susceptible to disease and damage. In some cases, this leads to bleeding that requires surgical intervention of targeted areas.

Surgeons make use of elongated medical devices such as catheters to navigate narrow passageways to a desired location to perform diagnostic and therapeutic procedures. Elongated medical devices can extend into a body from outside via an access point through various connected passageways to a target location. The elongated medical devices must meet a variety of requirements such as a desired length, a sufficiently small outer diameter to permit navigation of narrow body passageways, and sufficiently large inner diameter to permit delivery of the required functionality at the remote location. It is sometimes desired to perform electrosurgical procedures at the remote target location. In some cases, the target location is treated by balloon catheter dilation followed or accompanied by the application of electrical energy to perform the electrosurgical procedure. Heat cauterization is a commonly used hemostatic technique for bleeding wounds and can be accomplished through electrodes. Tissue is heated and coagulation is effected, which plugs the bleeding points and stops the bleeding.

Electrosurgical devices are configured for use with electrical energy, most commonly radio frequency (RF) energy, to cut tissue or to cauterize blood vessels by delivering electrosurgical energy to the tissue through the electrodes. With sufficiently high levels of electrical energy, the heat generated is sufficient to stop the bleeding from severed blood vessels. Current electrosurgical devices can cause the temperature of the tissue being treated to rise significantly higher than 100° Celsius, resulting is tissue desiccation, tissue sticking to the electrodes, tissue perforation, char formation and smoke generation. Peak tissue temperatures as a result of RF treatment of target tissue can be as high as 320° Celsius, and such high temperatures can be transmitted to adjacent tissue via thermal diffusion. Undesirable results of such transmission to adjacent tissue include unintended thermal damage to the tissue. Using saline coupled with RF electrical energy can help inhibit such undesirable effects. However, tissue desiccation and other undesirable results still occur when treatment of tissue occurs at temperatures exceeding 100° Celsius. Additionally, access and treatment of internal target locations can require multiple tools to support the cavity walls and perform electrosurgery and are not able to conform to a shape of a body cavity and contact the tissue to effectuate selective application of thermal energy to the tissue.

US 2013/184702 A1 relates to medical devices and methods for treating, reducing, or preventing stenosis using non-thermal irreversible electroporation.

### Summary

The invention is defined in the following claims; other embodiments, aspects or methods do not constitute a part of the invention. The electrosurgical device includes an inner catheter, an outer catheter, a balloon, and a plurality of tubular electrodes. The inner catheter extends along a longitudinal axis. The outer catheter includes a first section and a second section each disposed around the inner catheter. The balloon is disposed around the inner catheter and extends between the first and second sections of the outer catheter. The balloon is a tubular member having an outside surface and the plurality of tubular electrodes extend along the longitudinal axis adjacent to the outside surface of the balloon.

### Brief Description of the Drawings

Fig. 1 is a schematic illustration of a surgical system in accordance with principles of the present disclosure and with portions shown in block form;
Figs. 2A and 2B are exploded perspective views of a tip portion of an electrosurgical device useful with the system of Fig. 1 in accordance with aspects of the present disclosure;
Fig. 3A is an enlarged lengthwise cross-sectional view of the electrosurgical device of Fig. 2A;
Fig. 3B is an enlarged lengthwise cross-sectional view of the electrosurgical device of Fig. 2B;
Figs. 4A and 4B are enlarged cross-sectional views of the electrosurgical device in accordance with aspects of the present disclosure;
Fig. 4C is an enlarged cross-sectional view of the electrosurgical device of Fig. 2B; and
Figs. 5A-5D illustrate use of the electrosurgical device in performing a sinus procedure.

### Detailed Description

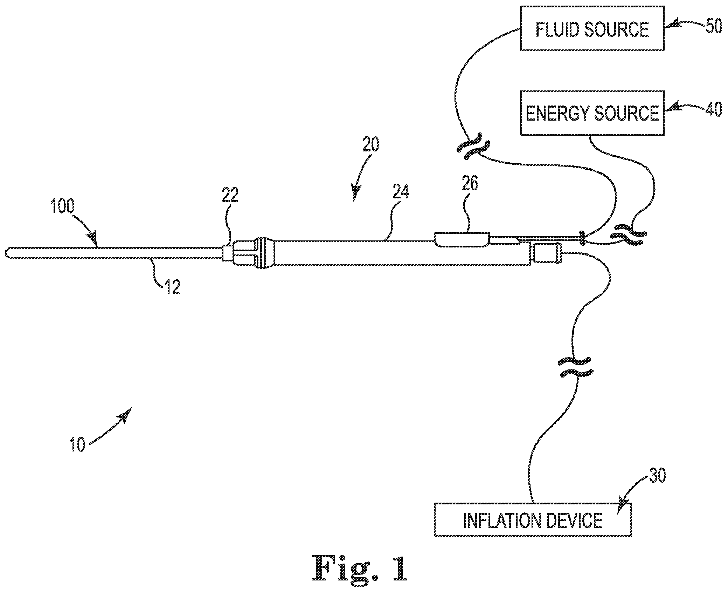

One embodiment of a surgical system 10 in accordance with principles of the present disclosure is illustrated in Fig. 1. The system 10 includes a surgical instrument 20 coupled to an inflation device 30, an energy source 40, and a fluid source 50. The inflation device 30, the energy source 40, and the fluid source 50 can be provided as stand-alone devices or can be included as part of the surgical system 10. The system 10 includes one or more electrosurgical devices 100 configured for coupling to, and use with, the instrument 20. In general terms, the electrosurgical device 100 is electronically connected to the energy source 40 and fluidly connected to the inflation device 30 and the fluid source 50 through the instrument 20. Once connected, the surgeon can perform an electrosurgical procedure on a patient as outlined below. Details on the various components of the system 10 and the electrosurgical device 100 used in the electrosurgical procedure are provided below.

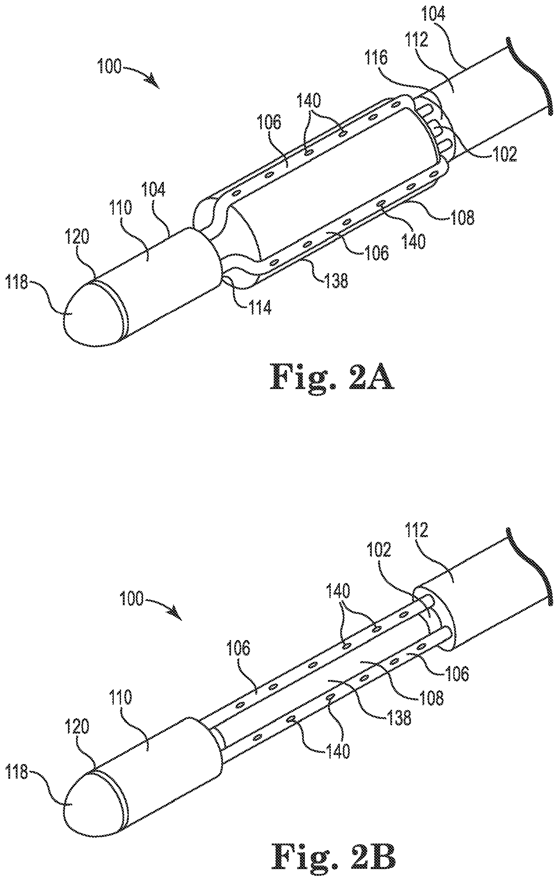

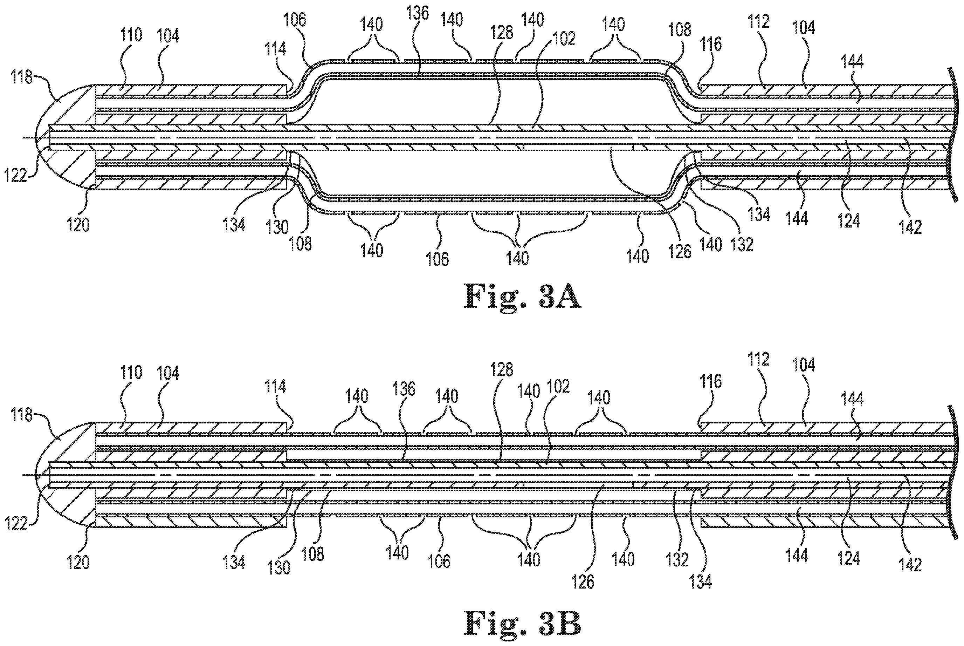

With the above in mind, perspective views of a tip portion of the electrosurgical device 100, in accordance with principles of the present disclosure, are shown in Figs. 2A and 2B. By way of example, Fig. 2A illustrates the device 100 in an expanded, or inflated, state and corresponds with the cross-sectional view illustrated in Fig. 3A. Fig. 2B illustrates the device 100 in an insertion, or deflated, state and corresponds with the cross-sectional view illustrated in Fig. 3B. The device 100 can be transitioned between a compact near linear configuration of the deflated state and the expanded state. The electrosurgical device 100 is constructed to expand to conform to and/or dilate a body lumen at the target site and simultaneously apply pressure and heat to tissue at the target site.

In general terms, the device 100 includes an inner catheter 102, an outer catheter 104, a plurality of electrodes 106, and a balloon 108. The outer catheter 104 includes a first section 110 and a second section 112. In one embodiment, a conical or dome shaped introducer 118 is attached at a distal end 120 of the first section 110. The introducer 118 provides for smooth insertion of the device 100 into the patient's body cavity or passageway.

With further reference to Figs. 3A and 3B, the inner catheter 102 extends distally from a trailing end (not shown) to an opposing, distal or leading end 122 along a longitudinal axis 142. The inner catheter 102 is a tubular body defining a passageway 124 and a port 126. The inner catheter 102 carries the balloon 108 and the outer catheter 104. The passageway 124 and the port 126 are configured to deliver pressurized fluid to the balloon 108 (from the fluid source 50) as well as remove the fluid from the balloon 108. In general terms, the delivery of pressurized fluid inflates the balloon 108 and removal of the fluid deflates the balloon 108. The pressurized fluid flows through the passageway 124 of the inner catheter 102 and enters the balloon 108 at the port 126. The port 124 can be circular, elongated, or any other appropriate shape. In one embodiment, more than one port 126 is included.

The balloon 108, or inflation member, is disposed around the inner catheter 102. In one embodiment, the balloon 108 is a tubular member. The balloon 108 is generally characterized as being more readily expandable than the inner catheter 102 or the outer catheter 104. The balloon 108 is can be formed of a variety of semi-compliant materials such as nylon, nylon derivatives, Pebax, polyurethane, or PET, for example. The balloon 108 is attached to the inner catheter 102 at a desired location or locations. In one embodiment, the balloon 108 is positioned proximal to the introducer 118. Alternatively, the balloon 108 can be positioned anywhere along the length of the inner catheter 102 or even in multiple locations along the inner catheter 102. The balloon 108 is mechanically or thermally attached and sealed to an outer surface 128 of the inner catheter 102 as indicated by a seal 134 at each of the opposing ends 130, 132 of the balloon 108. In one embodiment, the opposing ends 130, 132 are sealably attached to the inner catheter 102 with adhesive. The seal 134 is selected to be compatible with the materials of the inner catheter 102 and the balloon 108.

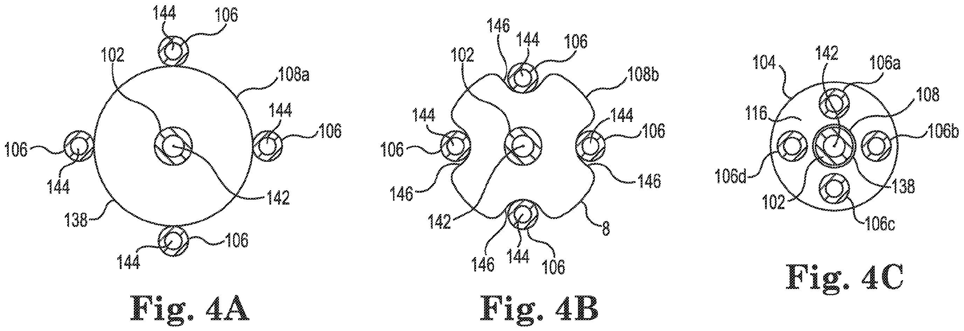

The balloon 108 is positioned along the inner catheter 102 such that an inflatable portion 136, extending between the opposing ends 130, 132, is free to inflate and deflate. When fully assembled, the inflatable portion 136 extends over the port 126 of the inner catheter 102. The balloon 108 has an outer surface 138. With additional reference to Fig. 4C, the balloon 108, in a deflated or contracted state, is generally sized and shaped in accordance with a size and shape of the inner catheter 102, thereby minimizing the outer profile of the device 100 along the balloon 108. The balloon 108 expands to, but not beyond, a preformed size and shape as reflected, for example, with the balloon 108a in Fig. 4A and with the balloon 108b in Fig. 4B when inflated at the expected operational inflation pressures. The maximum outer size of the balloon 108 upon inflation varies depending on the intended area of use (e.g., sinuses, esophagus, stomach, heart, etc.).

Returning to Figs. 2A-2B and 3A-3B, the outer catheter 104 is coaxial with, and is carried by, the inner catheter 102. The first and second sections 110, 112 have intermediate ends 114, 116, respectively, oriented toward one another. In one embodiment, the balloon 108 extends fully between the intermediate ends 114, 116 of the outer catheter 104. The first and second sections 110, 112 extend in opposite directions from the balloon 108 along the longitudinal axis 142. The outer catheter 104 is a generally cylindrical body and has an inner diameter correspondingly sized and configured to accommodate the outside diameter of the inner catheter 102. The inner diameter of the outer catheter 104 forms a main lumen extending the length of the outer catheter 104 that the inner catheter 102 is disposable through. In one embodiment, the inner diameter of the outer catheter 104 is slightly greater than an outer diameter of the inner catheter 102 allowing at least the second section 112 of the outer catheter 104 to be slidably disposed along the inner catheter 102. In one embodiment, where multiple balloons 108 are disposed along the inner catheter 102, second sections 112 on either end of the balloons 108 are slidably disposed along the inner catheter 102.

The inner and outer catheters 102, 104 are typically made of an electrically non-conductive material such as plastic or rubber. The inner catheter 102 and the plurality of electrodes 106 extend fully through the second section 112 and at least partially through first section 110 of the outer catheter 104. In some embodiments, the first section 110 terminates at the introducer 118 and the inner catheter 102 terminates at the leading end 122 within the introducer 118. Each of the plurality of electrodes 106 extends along the longitudinal axis 142 within a wall thickness formed between the inner and outer diameters of the outer catheter 104. The outer catheter 104 has a wall thickness between the inner diameter and an outer diameter that is sufficient to allow the electrodes 106 to pass within the wall and be electrically separated from each other. In one embodiment, the electrodes 106 are fixedly coupled to the first and second sections 110, 112 of the outer catheter 104. The electrodes 106 can be fixedly coupled to the outer catheter 104 with adhesive or other suitable means. Alternatively, the electrodes 106 are slidably disposed within lumens of the outer catheter 104 positioned around the main lumen to allow for longitudinal extension of the plurality of electrodes 106 disposed within the outer catheter 104, as discussed more below. The electrodes 106 can be slidably disposed within the first section 110, the second section 112, or both the first and second sections 110, 112.

The electrodes 106 are formed of elastic or shape memory material and have the ability to "remember" the shape given during original thermomechanical processing allowing the material to revert to the original shape when not extended beyond their elastic limit. The electrodes 106 are hypodermic tubing (i.e., hypotubes) made of spring tempered stainless steel, nickel-cobalt based alloy such as MP35 N or MP35 NLT, or nickel-titanium (nitinol) super elastic or shape memory, for example. Each electrode 106 is an elongated tubular member and includes a lumen 144 extending through the length of the electrode 106. The lumen 144 is fluidly coupled to the fluid source 50 (Fig. 1) and is configured as a fluid path for the saline or other suitable fluid.

The electrodes 106 are "weeping" electrodes in that the electrodes 106 include fluid ports 140 to dispense electrically charged fluid (e.g., saline). The fluid ports 140 can be either drilled or laser cut into the electrodes 106. The fluid ports 140 are formed along a length of the electrodes 106. When assembled, the fluid ports 140 are located alongside the balloon 108 between the first and second sections 110, 112 of the outer catheter 104. In one embodiment, the fluid ports 140 are facing radially outward relative to the balloon 108.

Each of the plurality of electrodes 106 extends from the first section 110, along the outer surface 138 of the balloon 108, and through the second section 112 of the outer catheter 104. The electrodes 106 extend along a longitudinal length of the inner catheter 102 and are arranged in a pattern about the circumference of the inner catheter 102. The electrodes 106 are spaced apart by a selected spacing and aligned along the longitudinal axis 142 of the inner catheter 102. With reference to the embodiments of Figs. 4A-4C, the electrodes 106 are spaced 90° from one another around the circumference of the inner catheter 102, although other spacing is also acceptable. For example, the electrodes 106 may be spaced 30°, 45°, or 60° from each other around the circumference.

The balloon 108 can be non-compliant (i.e., a certain shape) or compliant (i.e., conforms to the shape of the shaped area exposed to). Fig. 4A illustrates one example of a non-compliant balloon 108a. The plurality of electrodes 106 remain "proud" (i.e., outside the diameter of the balloon 108a). One example of a compliant balloon 108b is illustrated in Fig. 4B. The balloon 108b is shaped such that the plurality of electrodes 106 is at least partially recessed into longitudinal depressions 146 when the balloon 108b is fully inflated. The balloon 108b is sufficiently flexible to generally conform to a shape or curvature of the electrodes 106 within the expanded circumference of the balloon 108b yet sufficiently rigid enough to force the electrodes 106 away from the longitudinal axis 142 when inflated. As illustrated in Fig. 4C, whether the balloon 108 is compliant or non-compliant, in a non-inflated state, the balloon 108 has a diameter corresponding with the outer diameter of the inner catheter 102. As illustrated in Figs. 4A-4B, the balloon 108 in the expanded state extends a distance transverse of the longitudinal axis 142 greater than when in the deflated state illustrated in Fig. 4C.

The diameter of the electrodes 106, as well as the spacing between the electrodes 106, is suitable for providing hemostasis to the desired target area in which the device 100 is intended to be used. The spacing between the electrodes 106 determines the conveyance or non-conveyance of energy between electrodes 106. The spacing between the electrodes 106 is sufficiently close to allow conveyance of a given level of energy sufficient to cause hemostasis. The number of electrodes 106 is based, as least in part, on the size of the balloon 108 in the expanded state in order to have the desired spacing, and associated energy output, in the expanded state. The amount of energy to be delivered can also be a factor in the number and size of the electrodes 106. The energy transmitted to the electrodes 106 can be controlled to deliver a specific level of power to individual, a subset, or all of the electrodes 106.

With the above construction in mind, the plurality of electrodes 106 are configured to dispense saline, or other appropriate electrically conductive fluid, and deliver bipolar RF energy. In one embodiment, each of the electrodes 106 delivers an opposite energy to that of the immediately adjacent electrode. In other words, the plurality of electrodes 106 is bipolar with alternating electrodes 106 conducting either positive or negative current. For example, a positive current is delivered to the first and third electrodes 106a and 106c of Fig. 4C and a negative current is delivered to the second and fourth electrodes 106b and 106d. The saline is below boiling temperature (e.g., room temperature, body temperature, etc.) as it travels through each electrode 106. The saline dispensed from positively and negatively charged electrodes 106 intermingles as it is dispensed through the fluid ports 140, essentially causing a "shorting" of electrical energy. Boiling of the saline then occurs (i.e., a temperature of 100 degrees Celsius occurs).

The delivery of energy along with saline by the electrodes 106 provides therapeutic treatment, such as hemostasis, to the tissue within a target area. Coagulation, shrinkage of tissue, or sealing may also occur. The target area could be, for example, a portion of the sinuses, cardiovascular system, or gastrointestinal tract. The method includes controlling the delivery of radiofrequency energy into tissue by controlling energy delivery across the surface area of tissue within the target area and controlling delivery into the depth of tissue within the target area such that some volume of vessels of the tissue ceases to bleed.

In some examplary embodiments of the method, controlling the number of the electrodes 106 delivering radiofrequency energy and saline dispersement limits the portion of the target area that receives hemostasis. In other words, only a subset of the plurality of electrodes 106 may be employed in order to target a desired area. In this manner, only the electrodes 106 receiving energy and saline, and adjacent to a portion of the tissue target area, cause hemostasis. Thus, if only a subset of the plurality of electrodes 106 are employed, only the portion of tissue in the area of the subset of the electrodes 106, and not another portion of the tissue within the target area, is treated.

Electrosurgery methods in accordance with some examplary embodiments of the present disclosure can entail the surgeon receiving a single electrosurgical device 100 or a set of electrosurgical devices. The set, or kit, includes two or more devices each sized, shaped, and configured for insertion, accessing, and treating a different internal region of a patient. The surgeon determines the appropriate site or sites for treatment. Identifying the site(s) can be by endoscopic or other visualization and diagnostic methods known in the industry. The surgeon evaluates the area to be treated, considering the amount of energy to be delivered, the energy density, the duration of time over which energy is to be delivered, and the surface area to be treated and then selects the appropriate electrosurgical device 100. In one aspect, evaluation includes identifying the locale of the treatment site, including its dimensions, the multiplicity of sites if there is more than one site, and further identifying their locale and respective dimensions.

With continued reference to Fig. 1, in one embodiment, the energy source 40 is a radio frequency (RF) energy generator and the fluid source 50 is a saline source. The energy source 40 and the fluid source 50 supply energy and fluid, respectively, to the plurality of electrodes 106. The inner catheter 102 is fluidly open at the proximal end and fluidly connects with the inflation device 30 through the instrument 20. The inner catheter 102, the outer catheter 104, and each of the plurality of electrodes 106 of the device 100 is configured for coupling to the instrument 20 at a connector 22 to facilitate the fluid and electrical coupling to the device 100. In one embodiment, the outer catheter 104 is slidable relative to the instrument 20 when coupled. The instrument 20 includes a handle 24 and at least one switch 26 for selectively controlling power and/or fluid delivery to the electrosurgical device 100. The device 100, can be pre-bent along its length to a desired angle, linear, or configured to articulate. The device 100 can be partially or fully encased in a sheath 12. Once the device 100 is delivered to the target location, the sheath 12 can be retracted to expose the plurality of electrodes 106 extending along the balloon 108.

With the above construction in mind, use of the electrosurgical device 100 in treating internal bleeding of the patient entails coupling the device 100 electronically and fluidly to the instrument 20 which is electronically and fluidly coupled to the inflation device 30, the energy source 40, and the fluid source 50 of Fig. 1. In particular, the inner catheter 102 of the device 100 is fluidly connected to the inflation device 30 and the plurality of electrodes 106 are electronically and fluidly connected to the energy source 40 and the fluid source 50, respectively. The inflation device 30 is selectively fluidly connected to the electrosurgical device 100 and operates to effectuate inflation and deflation of the balloon 108. The inflation device 30 delivers pressurized fluid (e.g., air or water) through the inner catheter 102 for inflating the balloon 108. Depending on the area to be treated, a gas or liquid is used with the inflation device 30 to inflate the balloon 108 (e.g., air can be used if the treatment area is the esophagus, whereas water can be used if the treatment area is cardiovascular).

Once connected, the device 100 can be inserted into the patient's body cavity or internal passageway and maneuvered to position the balloon 108 of the device 100 at the target site. With reference back to Fig. 1, the device 100 can include the sheath 12. The sheath 12 is typically formed of a thin-walled plastic and is flexible. The sheath 12 surrounds the outer catheter 104, the inner catheter 102, the balloon 108, and the plurality of electrodes 106, during insertion and delivery of the device 100 to the target site, and serves to contain and isolate contaminants and mucus that may otherwise accumulate on the surfaces. The sheath 12 is retractable along the device 100 through use of an actuator (not shown) to expose the balloon 108 and the plurality of electrodes 106 disposed alongside the balloon 108 during inflation and delivery of RF energy and saline.

With the configuration discussed above, the device 100 can be extended into the patient's respiratory or other bodily tract in which a catheter is passable. The surgeon inserts the device 100 by initially inserting the leading end 122 and corresponding distal end 120 of the device 100 into the body cavity or passageway with the balloon 108 in the deflated state and the plurality of electrodes 106 in a first position with the electrodes 106 extending generally parallel to the longitudinal axis 142 of the inner catheter 102. The device 100 is pushed through the passageway and maneuvered to position the balloon 108 at the target site for electrosurgically treating the target site. Operation of the electrosurgical device 100 with respect to use in the frontal sinus of a patient is described in greater detail below. It is to be understood, however, that principles of the present disclosure are similarly provided in electrosurgical devices configured to access other sinuses, the cardiovascular system, and the gastrointestinal tract, for example.

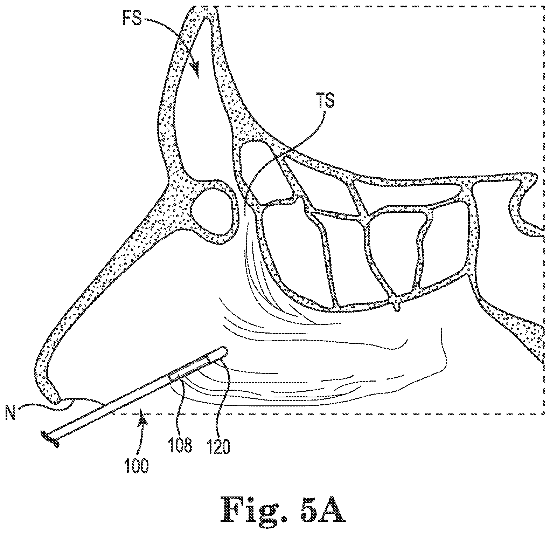

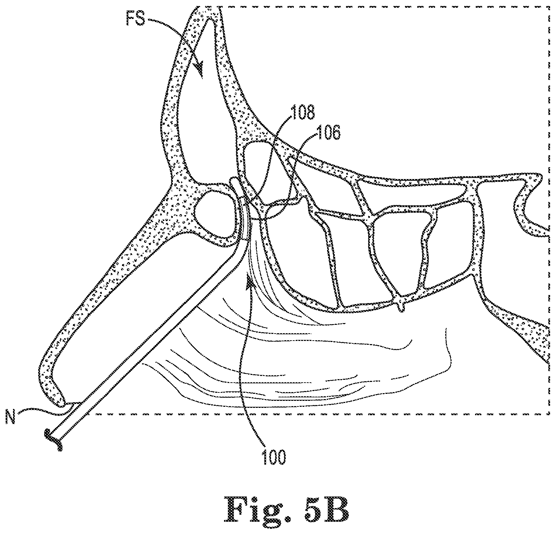

For example, Figs. 5A-5D illustrate various steps of a method of accessing and electrosurgically treating a frontal sinus FS using the device 100. With the surgeon grasping the instrument 22, the introducer 118 at the leading end 122 is initially introduced into the naris or nostril N (or other conventional approach) as shown in Fig. 5A. The device 100 is then further advanced through the patient's paranasal passageways, articulating or bending as required to access the targeted site TS and bringing the balloon 108 to the targeted site TS as illustrated in Fig. 5B. During the advancement of the device 100 through the patient's passageways, the balloon 108 is in a deflated state.

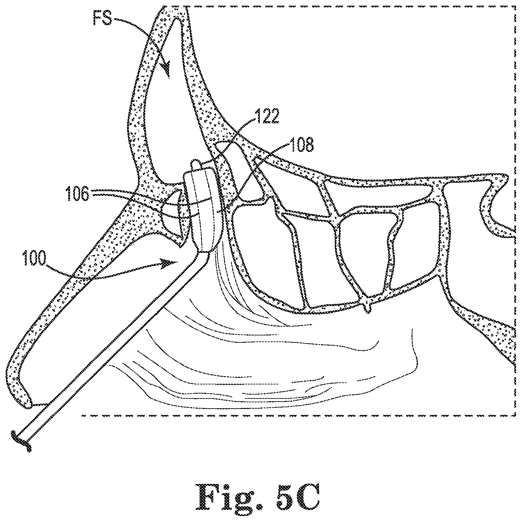

Upon placement of the device 100 at the target site and activation of the inflation device 30, the balloon 108 is inflated, as shown in Fig. 5C. As the inflation of the balloon 108 occurs, the patient's passageway can be expanded as desired. As the balloon 108 inflates, the plurality of electrodes 106 are expanded or extended outwardly (i.e., in a direction transverse from the longitudinal axis 142 a distance greater than when in the first position) to the second position by the force of the fluid filled balloon 108 pushing against the electrodes 106. In other words, the outward force of the balloon 108 expansion as it is filled with fluid presses the balloon 108 against the electrodes 106 and forces the electrodes 106 to correspondingly expand outward in a direction transverse from the longitudinal axis 142 to a second position to accommodate the expanded balloon 108. The length of the electrodes 106 disposed alongside the balloon 108 extends outwardly pressing against and widening the walls of the passageway. Additionally, for example, in the embodiment in which the second section 112 is slidably disposed along the inner catheter 102, as the electrodes 106 are extended or expanded outwardly from the first position to the second position, the second section 112 of the outer catheter 104 is drawn slidably along the inner catheter 102 toward the first section 110, bringing the two sections 110, 112 closer together.

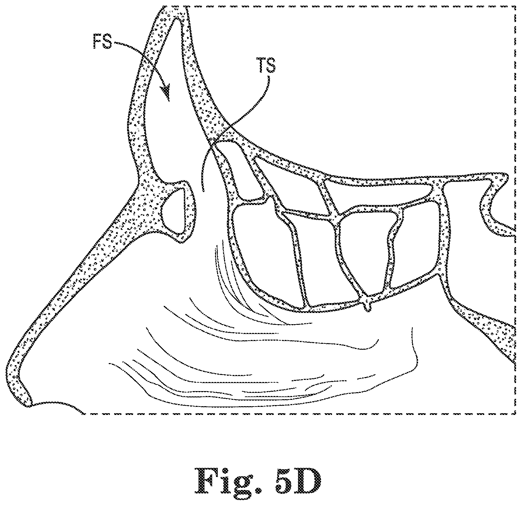

Once inflated, the energy source 40 and fluid source 50 illustrated in Fig. 1 are operated and RF energy and saline are delivered to the device 100. The surgeon operates the switch 26 of the instrument 20 (Fig. 1) to control the desired amount of RF energy delivered through all or a subset of the plurality of electrodes 106 and/or control the saline delivery. The saline enters the electrodes 106 at a temperature below 100° C and is dispensed from the fluid ports 140 of the electrodes 106 disposed along the length of the balloon 108 while remaining below 100°C. Depending on the area to be treated, all or a subset of the plurality of electrodes 106 are energized. The saline from bi-polar charged electrodes 106 intermingles and is heated by the RF energy to a temperature of 100° C. In some cases, the heated temperature is slightly above 100° C. After the appropriate amount of energy is delivered to cause hemostasis of the targeted tissue, delivery of RF energy and saline is terminated and the balloon 108 is deflated. In response to the balloon 108 deflation, the shape memory material of the plurality of electrodes 106 returns the electrodes 106 to the first position in the unextended state, and if applicable, and the second section 112 of the outer catheter 104 is returned to the original longitudinal extending position along the inner catheter 102. Following deflation of the balloon 108 and return of the electrodes 106 to the first position, the device 100 can be removed from the targeted site TS of the patient, as illustrate in Fig. 5D. In one embodiment, the saline is aspirated back through the electrodes 106 prior to removal of the device 100.

The surgeon can evaluate the treatment site(s) to determine if further treatment is needed. If appropriate, the above steps are repeated for further treatment. When multiple target areas are to be treated, the method may include the positioning, moving, inflating, and transmitting energy and saline steps to another target area without removing the electrosurgical device 100 from the patient.

In some embodiments, the device 100 of the present disclosure is a relatively inexpensive and disposable surgical tool (e.g., suitable for one-time use). Alternatively, in other constructions, the device can incorporate various structural features (e.g., materials, seals, etc.) that facilitate surgically-safe cleaning and sterilization (e.g., autoclave sterilization) and are re-usable. The device 100 and the instrument 20 are releasably mounted to one another. With these constructions, following the electrosurgical procedure, the device 100 is disengaged from the instrument 20, the instrument 20 is sterilized, and a new device 100 is assembled to the instrument 20 and the electronic and fluid connections carried by and through the instrument 20.

As described above, the device 100 reduces the time and cost associated with patient recovery. The device provides both dilation and therapeutic electrotherapy in a single device and procedure. Placing and maintaining the device 100 in the desired position and providing positive contact with the selected tissue enhances treatment. The ability to provide therapeutic therapy at 100° C reduces the opportunity to damage the balloon 108 and undesired results to the target tissue and surrounding tissue.

## Claims

1. An electrosurgical device (100) comprising:
an inner catheter (102) extending along a longitudinal axis (142);
an outer catheter (104) comprising a first section and a second section each disposed around the inner catheter (102);
a balloon (108) disposed around the inner catheter (102) and extending between the first and second sections of the outer catheter (104), the balloon (108) comprising a tubular member having an outside surface; and
a plurality of tubular electrodes (106) extending along the longitudinal axis adjacent to the outside surface of the balloon (108),
**characterised in that**:
at least one of the plurality of tubular electrodes includes a lumen (144) terminating at a fluid port (140).

2. The device of claim 1, wherein the plurality of tubular electrodes (106) each includes a plurality of fluid ports (140) disposed between the first and second sections of the outer catheter (104).

3. The device of claim 1, wherein the plurality of tubular electrodes (106) are expandable.

4. The device of claim 1, wherein plurality of tubular electrodes (106) are configured to deliver electrical energy and fluid.

5. The device of claim 1, wherein the balloon (108) includes opposing first and second ends sealably attached to the inner catheter (102).

6. The device of claim 1, wherein second section of the outer catheter (104) is slidably disposed around the inner catheter (102).

7. The device of claim 1, wherein the outer catheter (104) comprises an inner diameter, an outer diameter, and a wall thickness extending between the inner diameter and outer diameter, and wherein the plurality of electrodes (106) extend along the longitudinal axis within the wall thickness.

## Patentansprüche

1. Elektrochirurgische Vorrichtung (100), umfassend:
einen inneren Katheter (102), der sich entlang einer Längsachse (142) erstreckt;
einen äußeren Katheter (104), umfassend einen ersten Bereich und einen zweiten Bereich, die jeweils um den inneren Katheter (102) herum angeordnet sind;
einen Ballon (108), der um den inneren Katheter (102) herum angeordnet ist und sich zwischen dem ersten und zweiten Bereich des äußeren Katheters (104) erstreckt, wobei der Ballon (108) ein röhrenförmiges Element mit einer Außenfläche umfasst; und
mehrere röhrenförmige Elektroden (106), die sich entlang der Längsachse angrenzend an die Außenfläche des Ballons (108) erstrecken, **dadurch gekennzeichnet, dass**:
mindestens eine der mehreren röhrenförmigen Elektroden ein Lumen (144) beinhaltet, das an einem Fluidanschluss (140) endet.

2. Vorrichtung nach Anspruch 1, wobei die mehreren röhrenförmigen Elektroden (106) jeweils mehrere Fluidanschlüsse (140) beinhalten, die zwischen dem ersten und dem zweiten Bereich des äußeren Katheters (104) angeordnet sind.

3. Vorrichtung nach Anspruch 1, wobei die mehreren röhrenförmigen Elektroden (106) ausdehnbar sind.

4. Vorrichtung nach Anspruch 1, wobei mehrere röhrenförmige Elektroden (106) konfiguriert sind, um elektrische Energie und Flüssigkeit abzugeben.

5. Vorrichtung nach Anspruch 1, wobei der Ballon (108) gegenüberliegende erste und zweite Enden beinhaltet, die abdichtbar am inneren Katheter (102) befestigt sind.

6. Vorrichtung nach Anspruch 1, wobei der zweite Bereich des äußeren Katheters (104) verschiebbar um den inneren Katheter (102) herum angeordnet ist.

7. Vorrichtung nach Anspruch 1, wobei der äußere Katheter (104) einen Innendurchmesser, einen Außendurchmesser und eine Wanddicke umfasst, die sich zwischen dem Innendurchmesser und dem Außendurchmesser erstreckt, und wobei sich die mehreren Elektroden (106) entlang der Längsachse innerhalb der Wanddicke erstrecken.

## Revendications

1. Dispositif électrochirurgical (100) comprenant :
un cathéter interne (102) s'étendant le long d'un axe longitudinal (142) ;
un cathéter externe (104) comprenant une première section et une seconde section disposées chacune autour du cathéter interne (102) ;
un ballonnet (108) disposé autour du cathéter interne (102) et s'étendant entre les première et seconde sections du cathéter externe (104), le ballonnet (108) comprenant un élément tubulaire ayant une surface externe ; et
une pluralité d'électrodes tubulaires (106) s'étendant le long de l'axe longitudinal adjacent à la surface extérieure du ballonnet (108), **caractérisé en ce que** :
au moins une de la pluralité d'électrodes tubulaires comporte une lumière (144) se terminant au niveau d'un orifice pour fluide (140).

2. Dispositif selon la revendication 1, dans lequel la pluralité d'électrodes tubulaires (106) comporte chacune une pluralité d'orifices pour fluide (140) disposés entre les première et seconde sections du cathéter externe (104).

3. Dispositif selon la revendication 1, dans lequel la pluralité d'électrodes tubulaires (106) sont extensibles.

4. Dispositif selon la revendication 1, dans lequel une pluralité d'électrodes tubulaires (106) sont conçues pour fournir de l'énergie électrique et un fluide.

5. Dispositif selon la revendication 1, dans lequel le ballonnet (108) comporte des première et seconde extrémités opposées fixées de manière étanche au cathéter interne (102).

6. Dispositif selon la revendication 1, dans lequel la seconde section du cathéter externe (104) est disposée de manière coulissante autour du cathéter interne (102).

7. Dispositif selon la revendication 1, dans lequel le cathéter extérieur (104) comprend un diamètre intérieur, un diamètre extérieur et une épaisseur de paroi s'étendant entre le diamètre intérieur et le diamètre extérieur, et dans lequel la pluralité d'électrodes (106) s'étend le long de l'axe longitudinal à l'intérieur l'épaisseur de la paroi.
